# EUROPEAN PATENT SPECIFICATION

(11) **EP 1 335 700 B1**
(45) Date of publication and mention of the grant of the patent: **22.08.2007**
(21) Application number: 01980358.4
(22) Date of filing: 07.09.2001
(51) Int. Cl.: A61K 31/197, A61K 31/785, A61P 17/00

(54) **PHARMACEUTICAL COMPOSITION COMPRISING CHELATING/SEQUESTERING AGENTS AND THEIR DERMATOLOGICAL USE**
PHARMAZEUTISCHE ZUBEREITUNGEN, DIE CHELATBILDNER/SEQUESTRIERMITTEL ENTHALTEN UND DEREN DERMATOLOGISCHE VERWENDUNG
COMPOSITIONS PHARMACEUTIQUES COMPRENANT DES AGENTS CHELATANT/SEQUESTRANT ET LEUR UTILISATION DERMATLOGIQUE

(30) Priority: 11.09.2000 EP 00203131; 26.10.2000 EP 00203737
(43) Date of publication of application: 20.08.2003
(73) Proprietor: JOHNSON & JOHNSON GmbH, 40474 Düsseldorf (DE)
(72) Inventor: FERNANDEZ-KLEINLEIN, Elena, 40597 Düsseldorf (DE); HAUSER, Matthias, 53757 St. Augustin (DE); BIEHL, Petra, 41468 Neuss (DE); VON STETTEN, Otto, 52072 Aachen (DE)
(74) Representative: Weber-Bruls, Dorothée
(86) International application number: PCT/EP2001/010393
(87) International publication number: WO 2002/019981

(56) References cited:
- EP-A- 0 412 705
- WO-A-93/11737
- WO-A-99/62482
- DE-A- 19 907 014
- US-A- 5 540 863
- US-A- 5 635 167
- US-A- 5 977 053
- BABCOCK GOVE P.: "Webster's Third New International Dictionary" , MERRIAM-WEBSTER INC. , SPRINGFIELD, MASSACHUSETTS, U.S.A. XP002164871 see "1 chelate" and "2 chelate" page 383, middle column see "1 sequester" (definition 3) and "sequestrant" page 2071, right-hand column
- WENNINGER J.A. AND MCEWEN G.N. JR: "International Cosmetic Ingredient Dictionary and Handbook, Vol. 2" 1997 , THE COSMETIC, TOILETRY, AND FRAGRANCE ASSOCIATION , WASHINGTON, DC, U.S.A. XP002164872 page 1626, paragraph 2

## Description

This invention is concerned with pharmaceutical compositions comprising a chelating and a sequestering agent and optionally further components. The invention further concerns the preparation of said compositions and their use in the prevention or topical treatment of skin conditions such as eczema, irritation and skin dryness, as well the use of these compositions for making water more compatible with the skin.

### Background of the Invention

Water is omnipresent in the biosphere and is an integral and even the most abundant part of the of the human body, including its outer part, more in particular the skin. However, in certain circumstances water can have adverse effects on the human body, in particular on the skin. In fact the "beneficial window"of water is quite small compared to certain other chemicals, having a safety factor of about 4 to 5. The undesired effects of water, especially with regard to the skin, are largely dependent on water quality. The latter in turn depends on certain components that are present in water, in particular on the presence of ionic components. But even pure water is known to dry out healthy skin. Hard water on the other hand may lead to calcium precipitates on the skin that may cause drying, irritation and even atopic eczema. It has been demonstrated that the incidence of atopic eczema in school children is higher in areas with hard water compared with areas with soft water. Another problem associated with the usage of hard water is the decreased performance of cosmetic cleansers.

It is therefore an object of the present invention to prevent these undesired side effects of water to the skin and in particular to prevent the incidence of drying, irritation and atopic eczema. It is a further object to make water more compatible to the skin and to provide new compositions that lack the adverse effects relating to the use of water and in particular the use of hard water.

It is still a further object to provide compositions that prevent the formation of calcium precipitates, thus leading to a healthier skin, while at the same time improving the performance of cleansers such as e.g. soaps, shampoos, shower gels and the like.

EP-0884344 and EP-0884380 describe mild surfactant compositions comprising hydrophobic modified polyaspartic acid derivatives for cosmetic and cleansing use. These compositions are taught to be mild, but there is no mentioning of water quality or skin compatibility of water, only a description of antimicrobial action is given.

EP-0958811 describes modified polyaspartic acid in oil/water based emulsions for use in cosmetic applications such as day or night creams, moisturizing creams, body lotions and the like.

EP-0959093 describes a pigment paste containing derivatives of polyaspartic acid.

WO-00/49121 describes compositions for washing and cleaning, which compositions contain spray-dried polyaspartic acid and/or iminodisuccinates.

US-5,540,863 describes polyamino acids or their salts mixed with citric acid or its salts used to chelate calcium ions.

EP-0412705 describes a vehicle system for providing a desirable rheology of products formulated therewith, enhanced dispersion of actives therein, and improved deposition of actives therefrom. The vehicle system optionally contains a chelating agent.

WO-99/62482 discloses transparent cosmetic preparations containing a particular surfactant and a sequestering agent.

US-5,635,167 relates to a process for the removal of exogenous minerals which have become attached to hair being based on the usage of particular chelating agents.

WO-93/11737 discloses a composition for removing minerals from hair which composition comprises several agents and a chelating agent. Further disclosed is a synergistic combination of chelating agents.

US 5,977,053 is about a zeolite-free composition, comprising at least one polymer containing recurring succinyl units and/or soluble salts thereof and an iminodisuccinate. Detergents and cleaners containing this combination have been found to show improved biodegradability and technical performance, especially in terms of good primary washing power and improved secondary washing power in combination with reduced scale deposits and increased whiteness of the fabric.

The compositions of the present invention containing a combination of a chelating agent selected from the group consisting of iminodisuccinate salts, and the acid form thereof and a sequestering agent selected from the group consisting of a polyaspartic acid, and salts thereof, possess the desirable properties outlined above. More in particular it has been found that adding such a sequestering agent to such a chelating agent potentiates the water quality improving properties of chelating agents, in particular their water softening potential. Additionally thereto, it has been found that the topical application of the compositions of this invention shows beneficial effects on a number of skin conditions and therefore the present compositions can be used as agents to prevent adverse effects on the skin as well as to treat such skin conditions.

### Summary of the Invention

Thus in one aspect the present invention concerns a pharmaceutical composition comprising a pharmaceutically acceptable chelating agent and a pharmaceutically acceptable sequestering agent, wherein the chelating agent is an iminodisuccinate as defined below or a salt thereof, and the sequestering agent is a polyaspartic acid as defined below or a salt thereof.

The said composition may further contain appropriate ingredients suited for pharmaceutical formulations and, if desirable, further active ingredients.

In a particular aspect the composition is for topical use.

In a further aspect this invention concerns the use of a composition containing a combination of a chelating agent and a sequestering agent as defined above in aqueous pharmaceutical compositions for reducing water hardness. The invention further concerns the use of a pharmaceutical composition as defined above for making water more compatible to the skin.

The invention further provides the use of a composition containing a chelating and a sequestering agent as defined above to manufacture a pharmaceutical composition that has increased compatibility with the skin.

In still a further aspect, the invention provides the use of a composition containing a chelating and a sequestering agent as defined above to manufacture a pharmaceutical composition for treating a subject suffering from eczema, irritation and skin dryness, by topical administration of an effective amount of said composition.

Also disclosed is a process for manufacturing a pharmaceutical composition comprising a pharmaceutically acceptable chelating agent and a pharmaceutically acceptable sequestering agent and optional other ingredients, characterized by mixing the said chelating agent and said sequestering agent, and said optional other ingredients.

### Detailed Description of the Invention

The present invention concerns pharmaceutical compositions comprising a pharmaceutically acceptable chelating agent and a pharmaceutically acceptable sequestering agent, wherein the chelating agent is an iminodisuccinate as defined below or a salt thereof, and the sequestering agent is a polyaspartic and as defined below or a salt thereof.

Particular salts are those which are pharmaceutically acceptable.

The compositions according to the present invention may contain one or more chelating agents and one or more sequestering agents. The term 'chelating agent' as used herein is meant to refer to one or a plurality of chelating agents and similarly the term 'sequestering agent' is meant to refer to one or a plurality of sequestering agents.

The terms 'salts' as used herein refer to pharmaceutically acceptable base-addition salt forms of the components mentioned above. Pharmaceutically acceptable salts are those which are acceptable for topical pharmaceutical use, said salts preferably being non-toxic. Said base-addition salt forms in particular are alkali metal, e.g. sodium or potassium, or ammonium, or substituted ammonium salt forms or benzathine, N-methyl-D-glucamine, hydrabamine salts, or salts with amino acids such as, for example, arginine, lysine Substituted ammonium as used herein refers to any non-toxic substituted ammonium ion known or used in the art as a salt former and comprises mono-. di-, and in particular trior quaternary substituted ammonium salts, including mono- or polycyclic systems. Substituents on ammonium for example are alkyl, cycloalkyl, alkenyl, substituted alkyl such as hydroxyalkyl, alkyloxyalkyl, (cycloalkyl)alkyl, arylalkyl such as benzyl.

As used herein 'alkyl' refers to a saturated hydrocarbon in particular a hydrocarbon radical having one to about 20 carbon atoms, in particular 1 to about 8 carbon atoms, straight or branch chained. 'Alkyl' in hydroxyalkyl or alkoxylalkyl preferably has 2 to 6, more preferably 2 to 4 carbon atoms. 'Alkenyl' similarly refers to a hydrocarbon having one or more double bonds, e.g. one, two or three double bonds.An alkenyl radical may be straight or branch chained and may have 2 to 20, in particular 3 to 8 carbon atoms. Cycloalkyl may have further alkyl substituents, in particular alkyl having 1 to 6 carbon atoms, and has from 5 to 8 ring atoms.

One or more of the above base-originating cations may be present, either the same or different.

As used herein, the term 'iminodisuccinate' refers to compounds which can be structurally represented by the following formula: 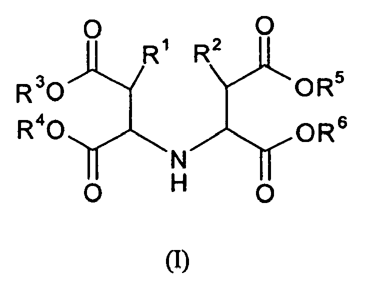 wherein:
R¹ and R² independently from one another represent hydrogen or hydroxy;
R³, R⁴, R⁵ and R⁶ independently from one another represent hydrogen or a suitable cation such as for example the alkali metal or ammonium cations mentioned hereinabove in relation to the term 'salts'.

In a particular embodiment the term 'imidosuccinate' as used herein refers to the compound N-(1,2-dicarboxyethyl)-aspartic acid and its salt forms. Salt forms of said compound are meant to comprise salts wherein one, two, three or four of the carboxyl hydrogens have been replaced by a suitable cation, or mixtures thereof. Examples are mono-, di-, tri- or tetra- sodium or potassium, or ammonium N-(1,2-dicarboxyethyl)-aspartic acid salts, wherein ammonium is as defined hereinabove.

The term 'polyaspartic acid' refers to polymers made of aspartic acid monomeric units. In particular polysapartic acid comprises polymers built up of one or more different aspartic acid units being represented by the following structural formulae: 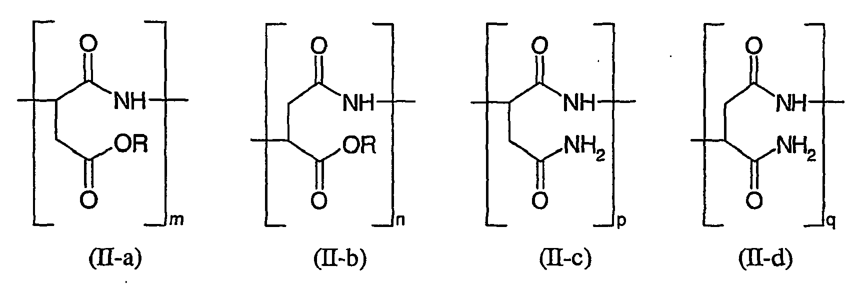 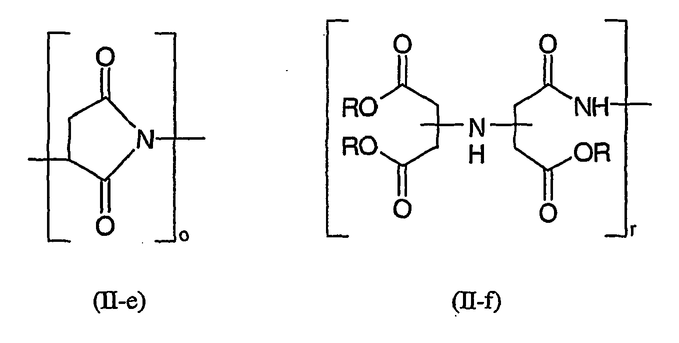 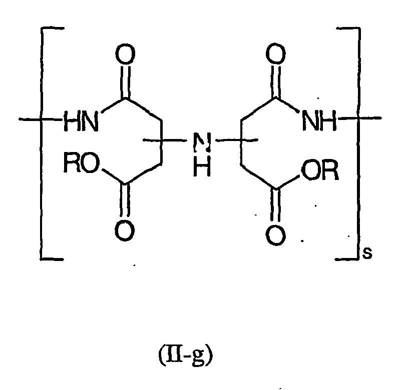 wherein:
each R independently represents hydrogen or a suitable cation such as for example the alkali metal or ammonium cations mentioned hereinabove in relation to the term 'salts';
m, n, o, p, q, r and s independently from one another are 0 or an integer up to 300, preferably up to 100, more preferably up to 50, whereby the sum of m + n + o + p + q + r + s is in the range of 4 to 300, preferably 4 to 100, more preferably 4 to 50, still more preferably between 10 to 40, most preferably between 15 and 30.

Preferred polymers are those wherein:
p and q independently are 0 or an integer from 1 to 10;
r is 0 or the integer 1 or 2;
s is 0 or an integer from 1 to 10.

Other polyaspartic acids that can be used are those, as defined hereinabove, wherein part of the above referred monomeric units is replaced by analogs such as:
malic acid units of formula 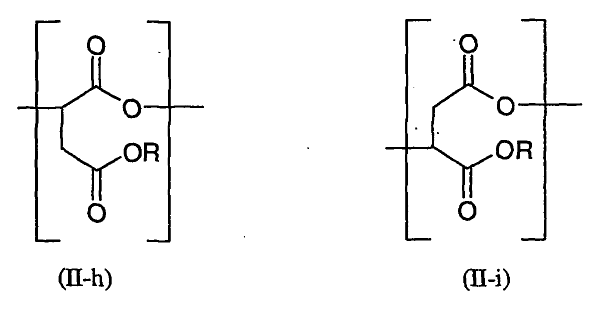
wherein R is as defined above;
maleic acid and and fumaric acid units of formula

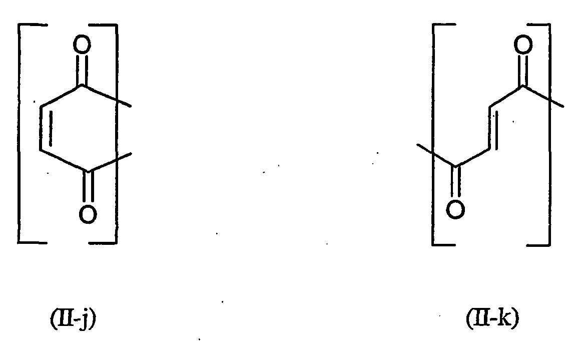

In particular up to 10 monomeric units per polymeric molecule; more particularly up to 5 units, e.g. 1 or 2 units, may be replaced by any of the above malic, maleic or fumaric residues.

Preferably, the polyaspartic acid used in the formulations of the invention has a mean molar mass in the range of 500 to 100,000 g/mole, in particular in the range of 1000 to 50,000 g/mole, more in particular between 1000 and 30,000. Preferred is a mean molar mass in the range of 800 to 5000 g/mol, more preferably in the range of 1000 to 4000 g/mol, still more preferably in the range of 1500-3000 g/mol.

A particularly preferred polyaspartic acid or a salt thereof can be represented by the following structural formula: 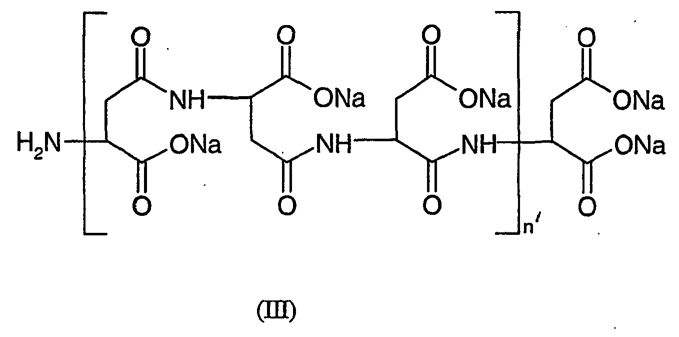 wherein n' is an integer in the range of 1 to 300, preferably 1 to 100, more preferably 1 to 50, or 1 to 40, or 4 to 30, most preferably between 15 and 30.

In the compounds of formula (III) some or all of the sodium cations may be / C replaced by one or more cations selected from hydrogen or the other alkalimetal cations, in particular potassium or ammonium or substituted ammonium.

Also particularly preferred are the potassium analogs of said particularly polyaspartic acid salt, or the acid itself. Of further interest is the above particularly preferred polyaspartic sodium or potassium salt wherein one or more of the sodium or potassium cations are replaced by hydrogen ions, more in particular wherein one or two sodium or potassium cations per monomeric unit are replaced by hydrogen ions.

The concentration of the chelating and sequestering agent in the compositions of the invention may vary but will in general be such that a sufficient reduction of the concentration of free calcium and/or magnesium ions and/or other metal ions causing problems like iron, copper, manganese is obtained. In particular the concentration of the chelating agent will be from 0,1-95 %, preferably from 0,2 - 90 %, more preferably from 0,4 - 85 % w/w relative to the total weight of the composition. In particular the concentration of the sequestering agent will be from 0.1-45 %, preferably from 0,1 - 40 %, more preferably from 0,2 - 35 % w/w relative to the total weight of the composition.

The w/w ratio of the chelating and sequestering agent preferably is in the range of from 20 : 1 to 1 : 20, more preferably from 10 : 1 to 1 : 10 most preferably from 5 : 1 to 1:5.

In the compositions of the invention, the w/w ratio of the chelating and sequestering agents may vary as described above, but also formulations having a low content of sequestering agent, i.e. where the w/w ratio of the chelating agent to the sequestering agent is 10 : 1 or higher, or 20 : 1 or higher, e.g. in the range of 20 : 1 to 25 : 1, or 25 : 1 to 30 : 1, or even 30 : 1 to 40 : 1 or to 50 : 1 are desirable combinations forming compositions with the attractive properties described herein.

The pharmaceutical compositions according to the present invention may additionally contain further ingredients or additives such as surfactants, emulsifiers, consistency factors, conditioners, emollients, skin caring ingredients, moisturizers, thickeners, tablet disintegrants, glidants, lubricants, fillers, binding agents, anti-oxidants, preservatives, active ingredient, in particular dermatologically active ingredients, fragrances. Active ingredients as mentioned herein comprise, for example, anti-inflammatories, anti-bacterials, anti-fungals agents. Active ingredients suited for topical applications are particularly preferred.

Suitable surfactants comprise:
alkyl sulfates e.g. sodium lauryl sulfate, ammonium lauryl sulfate, sodium cetearyl sulfate,
alkyl sulfoacetates e.g. sodium lauryl sulfoacetate,
alkyl ether sulfates e.g. sodium laureth sulfate, sodium trideceth sulfate, sodium oleth sulfate, ammonium laureth sulfate,
alkyl ether sulfosuccinates e.g. disodium laureth sulfosuccinate,
alkyl glycosides e.g. decyl glucoside, lauryl glucoside,
alkyl isethionates,
amphoterics e.g. cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium salts of the aforementioned amphoterics, capryl/capramidopropyl betaine, undecylenamidopropyl betaine, lauramidopropyl betaine and
fatty alcohol polyglycol ethers.

Suitable emulsifiers are e.g. anionics as salts of fatty acids e.g. sodium stearate or sodium palmitate, organic soaps e.g. mono-, di- or triethanolaminoleat, sulfated or sulfonated compounds e.g. sodium lauryl sulfate or sodium cetyl sulfonate, saponines, lamepones; cationics as quaternary ammonium salts; nonionics as fatty alcohols, fatty acid ester with saturated or unsaturated fatty acids, polyoxyethylenesters or polyoxyethylenethers of fatty acids, polymers from ethylene oxide and propylene oxide or propylene glycol, amphotherics as phosphatides, proteines as gelatine, casein alkylamidobetaines, alkyl betaines and amphoglycinates, alkyl phosphates, alkylpolyoxyethylene phoaphates or the corresponding acids, silicone derivatives, e.g. alkyl dimethiconecopolyol.

Suitable consistency factors are e.g. fatty alcohols or their mixtures with fatty acid esters, e.g. acetylated lanolin alcohol, aluminum stearates, carbomer, cetyl alcohol, glyceryl oleate, glyceryl stearate, glyceryl stearate (and) PEG 100 stearate, magnesium stearate, magnesium sulfate, oleic acid, stearic acid, stearyl alcohol, myristyl myristate, isopropyl palmitate, beeswax and synthetic equivalents thereof, carbomers. Suitable conditioners are e.g. alkylamido ammonium lactate, cetrimonium chloride and distearoylethyl hydroxyethylmonium methosulfate and cetearyl alcohol, cetyl dimethicone, cetyl ricinoleate, dimethicone, laureth-23, laureth-4, polydecene, retinyl pahnitate, quaternised protein hydrolysates, quaternised cellulose and starch derivatives, quaternised copolymers of acrylic or methacrylic acid or salts, quaternised silicone derivatives.

Suitable emollients are e.g. cetearyl isononanoate, cetearyl octanoate, decyl oleate, isooctyl stearate, coco caprylate/caprate, ethylhexyl hydroxystearate, ethylhexyl isononanoate, isopropyl isostearate, isopropyl myristate, oleyl oleate, hexyl laurate, paraffinum liquidum, PEG-75 lanolin, PEG-7 glyceryl cocoate, petrolatum, ozokerite, cyclomethicone, dimethicone, dimethicone copolyol, dicaprylyl ether, Butyrospermum parkii, Buxus chinensis, canola, Carnauba cera, copernicia cerifera, Oenothera biennis, Elaeis guineensis, Prunus dulcis, squalane, Zea mays, glycine soja, Helianthus annuus, lanolin, hydrogenated castor oil, hydrogenated coconut oil, hydrogenated polyisobutene, sucrose cocoate, stearoxy dimethicone, lanolin alcohol, isohexadecane.

Suitable skin caring ingredients are e.g. plant extracts, bisabolol, antiinflammatory agents, urea, allantoin, panthenol and panthenol derivatives, phytantriol, vitamines A, E, C, D, ceramides of animal or plant origin, lecithins.

Suitable moisturizers are e.g. butylene glycol, cetyl alcohol, dimethicone, dimyristyl tartrate, glucose, glycereth-26, glycerin, glyceryl stearate, hydrolyzed milk protein, lactic acid, lactose and other sugars, laureth-8, lecithin, octoxyglycerin, PEG-12, PEG-135, PEG-150, PEG-20, PEG-8, pentylene glycol, hexylene glycol, phytantriol, polyquaternium-39, PPQ-20 methyl glucose ether, propylene glycol, sodium hyaluronate, sodium lactate, sodium PCA, sorbitol, succinoglycan, synthetic beeswax, tri-C14-15 alkyl citrate, starch.

Suitable thickeners are e.g. acrylates/steareth-20 methacrylate copolymer, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl silylate, xanthan gum, hydrogenated butylene/ethylene/styrene copolymer.

Suitable tablet disintegrants are e.g. starch, sodium starch glycolate, crospovidone, sodium croscarmellose, alginic acid and alginates, calcium carbonate, sodium bicarbonate, magnesium peroxide, amylose, sodium and calcium carboxymethylcellulose, polyvinylpyrrolidone, cetyl alcohol, glycerin monostearate, lactose.

Suitable fillers are e.g. tricalciumphosphate, starch, sugar, lactic sugar, glucose, sodium chloride.

Suitable glidants are e.g. starch, magnesium stearate, calcium stearate, silica (Aerosil^{®}/Cabosil^{®}), amylose, talcum, tricalciumphosphate, magnesium oxide, calcium and magnesium carbonate, trimagnesium phosphate, tetrasodium diphosphate, silicium dioxide, aluminium silicate, kaolin, calcium and magnesium silicate, bentonite, magnesium trisilicate, calcium gluconate, salts of myristic, palmitic or stearic acid, sodium aluminium silicate.

Suitable lubricants are e.g. adipic acid, fumaric acid and its salts, benzoic acid and its salts, glycerine triacetate, sodium or magnesium lauryl sulfate, magnesium stearate, solid polyethylenglycol, polyvinylpyrrolidone, boric acid, monolaurate or-palmitate, myristyl alcohol, cetyl alcohol, cetylstearyl alcohol, talcum, calcium or magnesium salts of higher fatty acids, mono-, di- or triglycerides of higher fatty acids, polytetrafluorethylene.

Suitable binding agents are e.g. starch, carboxymethylcellulose, polyvinylpyrrolidone, gummi arabicum, calcium sulphate, calcium phosphate, veegum, sorbitol, sodium alginate, gelatine.

Suitable anti-oxidants are e.g. sulfites, e.g. sodium sulfite, tocopherol or derivates thereof, ascorbic acid or derivates thereof, citric acid, propyl gallate, chitosan glycolate, cysteine, N-acetyl cysteine plus zinc sulfate, thiosulfates, e.g. sodium thiosulfate, polyphenoles.

Suitable preservatives are e.g. benzyl alcohol, chloroxylenol, (sodium) methylparaben, (sodium) ethylparaben, (sodium) propylparaben, (sodium) butylparaben, phenoxyethanol, methylchloroisothiazolinone, methylisothiazolinone, sodium benzoate, chlorhexidine digluconate methyldibromoglutaronitrile, sodium borate, 5-bromo-5-nitro-1,3-dioxane, alcohol, benzoic acid, dehydroacetic acid, diazolidinyl urea, dichlorobenzyl alcohol, glucose oxidase, hexamidine diisethionate, imidazolidinyl urea, iodopropynyl butylcarbamate, isobutylparaben, isopropylparaben, lactoperoxidase, magnesium nitrate, PEG-4 laurate, phenethyl alcohol, polyaminopropyl biguanide, potassium sorbate, propylene glycol, pyridoxine HCl, quaternium-15, sorbic acid, triclosan and mixtures thereof.

The compositions may further contain active ingredients, e.g. anti-microbials such as complexes of PVP and hydrogen peroxide, anti-inflammatories, plant extracts, bisabolol, panthenol, tocopherol, actives for anti-stinging, anti-irritants, anti-dandruffs, for anti-ageing e.g. retinol, melibiose. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glycerin, glyceryl laurate, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, lactis proteinum, lactose, Lawsonia inermis, linalool, Linum usitatissimum, lysine, Magnesium aspartate, magnifera indica, Malva sylvestris, mannitol, mel, Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, prunus armeniaca, Prunus persica, retinyl palmitate, Ricinus communis, Rosa canina, Rosmarinus officinalis, rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talcum, thymus vulgaris, Tilia cordata, tocopherol, tocopheryl acetate, trideceth-9, Triticum vulgare, tyrosine, undecylenoyl glycine, urea, Vaccinium myrtillus, valine, zinc oxide, zinc sulfate.

The compositions of the present invention are generally prepared by mixing the appropriate chelating and sequestering agents and other ingredients. Subsequently other ingredients, such as perfumes, may be added.

A number of the properties of the present compositions and the results of their use according the present invention, as described herein, are due to the combination of a chelating and a sequestering agent, and are either more beneficial than in compositions with only one of said agents.

The compositions of the present invention comprising imidodisuccinate or salts thereof, and polyaspartic acid and salts thereof, as defined or described herein, are particular attractive due to their increased biodegredability which makes them environmentally more acceptable.

The compositions of the invention may take the form of a stand alone product, i.e. an additive to baths, shampoos, or a combination product. They may be solid, semi-solid or liquid, for example they can be solutions, emulsions, pastes, powders, granulates, tablets, effervescent tablets, gels, wet or dry wipes

The pharmaceutical preparations of the invention can be used to improve water quality, either in other aqueous formulations, in particular for topical application, or in aquous media for sanitary applications such as bathing water.

Therefore in a further aspect the invention provides a method of improving water quality in topical pharmaceutical compositions or in aqueous media for sanitary applications, said method comprising adding to said pharmaceutical or said aqueous media a composition comprising a pharmaceutically acceptable chelating agent and a pharmaceutically acceptable sequestering agent.

Or similarly, the invention provides the use of a composition according to the present invention to improve water quality in topical pharmaceutical compositions or in aqueous media for sanitary applications.

As used herein water quality refers to water hardness, smell, appearance (colour), and the presence of solids, the latter including the presence solids upon contact with soap, detergents, shampoo and the like.

In view of these properties compositions like e.g. bath or shower gels, are particularly attractive. Of particular interest are compositions for use with babies, infants or small children.

The compositions of the present invention show activity against a number of skin conditions such as eczema, irritation and skin dryness or prevent them when used in conjunction with cleansing before these conditions arise. These skin conditions may be treated by topically administering an appropriate composition according to this inventions such as an ointment, salve, lotion. Or alternatively the compositions may be added to water in which the affected is immerged or as a bathing or showering additive. They especially render the water used for cleansing more compatible to skin.

In the light of this activity, the invention further is concerned with the use of a combination of a chelating and a sequestring agent, or a pharmaceutical composition comprising a chelating and a sequestering agent as defined above for the manufacture of a medicament for topically treating a skin condition such as eczema, irritation and skin dryness.

The following examples are meant to illustrate the invention. As used herein, percentages of chelating or sequestering agents are w/w percentages of the solid material in water.

### Examples

### Example 1: Powder

| **Ingredient** | **weight [%]** |
|---|---|
| Sodium bicarbonate | 41.67 |
| Citric acid | 52.38 |
| Sodium polyaspartic acid (82 %) | 1.19 |
| Imino disuccinate (76 %) | 3.57 |
| Tapioca starch | 0.60 |
| Perfume | 0.60 |

Blend all ingredients under dry conditions.

### Example 2: Powder (comparative example)

| | |
|---|---|
| Sodium bicarbonate | 41.67 |
| Citric acid | 52.38 |
| Sodium polyaspartic acid (82 %) | 1.19 |
| Nitrilotriacetic trisodium salt | 3.57 |
| Tapioca starch | 0.60 |
| Perfume | 0.60 |

Blend all ingredients under dry conditions.

### Example 3: Powder with surfactant

| | |
|---|---|
| Sodium bicarbonate | 37.23 |
| Citric acid | 46.81 |
| Sodium lauryl sulfoacetate | 10.64 |
| Sodium polyaspartic acid (82 %) | 1.06 |
| Imino disuccinate (76 %) | 3.19 |
| Tapioca starch | 0.53 |
| Perfume | 0.53 |

Blend all ingredients under dry conditions.

### Example 4: Powder with surfactant (comparative example)

| | |
|---|---|
| Sodium bicarbonate | 37.23 |
| Citric acid | 46.81 |
| Sodium lauryl sulfoacetate | 10.64 |
| Sodium polyaspartic acid (82 %) | 1.06 |
| EDTA (trisodium salt) | 3.19 |
| Tapioca starch | 0.53 |
| Perfume | 0.53 |

Blend all ingredients under dry conditions.

### Example 5: Liquid

| | |
|---|---|
| Sodium polyaspartic acid (40 %) | 0.50 |
| Imino disuccinate (34 %) | 6.00 |
| Extrapone Camomile special® | 1.00 |
| Sodium sulphite | 0.50 |
| Phenoxyethanol | 1.00 |
| Aqua | 87.70 |
| Perfume | 0.30 |
| Polysorbate | 2.00 |
| Lactic acid | 1.00 |

Mix ingredients stirring into water. Add perfume premixed with polysorbate. Adjust pH to 6-7.

### Example 6: Liquid (comparative example)

| | |
|---|---|
| Sodium polyaspartic acid (40 %) | 0.50 |
| HEDTA (trisodum salt) | 6.00 |
| Extrapone Camomile special® | 1.00 |
| Sodium sulphite | 0.50 |
| Phenoxyethanol | 1.00 |
| Aqua | 87.70 |
| Perfume | 0.30 |
| Polysorbate | 2.00 |
| Lactic acid | 1.00 |

Mix ingredients stirring into water. Add perfume premixed with polysorbate. Adjust pH to 6-7.

### Example 7: Liquid with anti-microbial

| | |
|---|---|
| Sodium polyaspartic acid (40%) | 3.50 |
| Imino disuccinate (34%) | 7.00 |
| Extrapone Camomile special® | 3.00 |
| Polyquaternium-47 | 1.00 |
| PVP-H₂O₂ | 1.00 |
| Phenoxyethanol | 0.80 |
| Aqua | 77.85 |
| Lactic acid | 1.00 |
| Polysorbate | 4.00 |
| Perfume | 0.35 |
| Dye (0,1 %) | 0.50 |

Stir ingredients into water phase. Add perfume premixed with polysorbate. Adjust pH to 6-7.

### Example 8: Liquid with anti-microbial (comparative example)

| | |
|---|---|
| Carboxymethylcellulose | 3.50 |
| DTPA (trisodium salt) | 7.00 |
| Extrapone Camomile special® | 3.00 |
| Polyquaternium-47 | 1.00 |
| PVP-H₂O₂ | 1.00 |
| Phenoxyethanol | 0.80 |
| Aqua | 77.85 |
| Lactic acid | 1.00 |
| Polysorbate | 4.00 |
| Perfume | 0.35 |
| Dye (0,1 %) | 0.50 |

Stir ingredients into water phase. Add perfume premixed with polysorbate. Adjust pH to 6-7.

### Example 9: Liquid as bath product

| | |
|---|---|
| Sodium polyaspartic acid (40%) | 5.00 |
| Imino disuccinate (34%) | 5.00 |
| Sodium laureth sulphate | 4.30 |
| Decyl glucoside | 4.00 |
| Cocamidopropyl betaine | 7.90 |
| Lauryl betaine | 2.30 |
| Phenoxyethanol | 0.80 |
| Sodium propylparaben | 0.11 |
| Sodium methylparaben | 0.17 |
| Sodium butylparaben | 0.06 |
| Aqua | 69.86 |
| Perfume | 0.50 |

Stir ingredients into water phase. Add perfume premixed with polysorbate. Adjust pH to 6-7.

### Example 10: Tablets

| | |
|---|---|
| Sodium bicarbonate | 40.00 |
| Citric acid | 32.00 |
| Magnesium stearate | 1.00 |
| Sodium polyaspartic acid (82 %) | 5.00 |
| Polyethylenglycol | 5.00 |
| Imino disuccinate (76 %) | 13.00 |
| Sodium carboxymethyl cellulose | 3.00 |
| Silica | 1.00 |

Mix all ingredients thoroughly and press the mixture to tablets.

### Example 11: Tablets (comparative example)

| | |
|---|---|
| Sodium bicarbonate | 40.00 |
| Citric acid | 32.00 |
| Magnesium stearate | 1.00 |
| Sodium polyaspartic acid (82 %) | 5.00 |
| Polyethylenglycol | 5.00 |
| EDTA (trisodium salt) | 13.00 |
| Sodium carboxymethyl cellulose | 3.00 |
| Silica | 1.00 |

Mix all ingredients thoroughly and press the mixture to tablets.

### Example 12: Tablets with surfactant

| | |
|---|---|
| Sodium bicarbonate | 37.23 |
| Citric acid | 46.81 |
| Sodium lauryl sulfoacetate | 10.64 |
| Sodium polyaspartic acid (82 %) | 1.06 |
| Imino disuccinate (76 %) | 3.19 |
| Tapioca starch | 0.53 |
| Perfume | 0.53 |

Mix all ingredients thoroughly with the premix perfume and tapioca starch. Then compress the mixture to tablets.

### Example 13: Tablets with surfactant (comparative example)

| | |
|---|---|
| Sodium bicarbonate | 37.23 |
| Citric acid | 46.81 |
| Sodium lauryl sulfoacetate | 10.64 |
| Sodium polyaspartic acid (82 %) | 1.06 |
| NTA (trisodium salt) | 3.19 |
| Tapioca starch | 0.53 |
| Perfume | 0.53 |

Mix all ingredients thoroughly with the premix perfume and tapioca starch. Then compress the mixture to tablets.

## Claims

1. A pharmaceutical composition comprising
a cosmetically or a pharmaceutically acceptable chelating agent selected from the group consisting of iminodisuccinate salts and the acid form thereof; and a cosmetically or a pharmaceutically acceptable sequestering agent selected from the group consisting of polyaspartic acid and its salts, wherein the chelating agent is a compound which can be structurally represented by the formula: 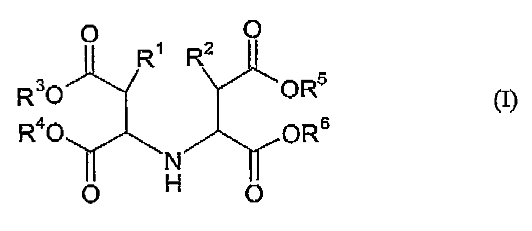 wherein:
R¹ and R² independently from one another represent hydrogen or hydroxy;
R³, R⁴, R⁵ and R⁶ independently from one another represent hydrogen or an alkali metal or ammonium or substituted ammonium cation;
and the sequestering agent is a polymer built up of one or more aspartic acid units which can be represented by the following structural formulae: 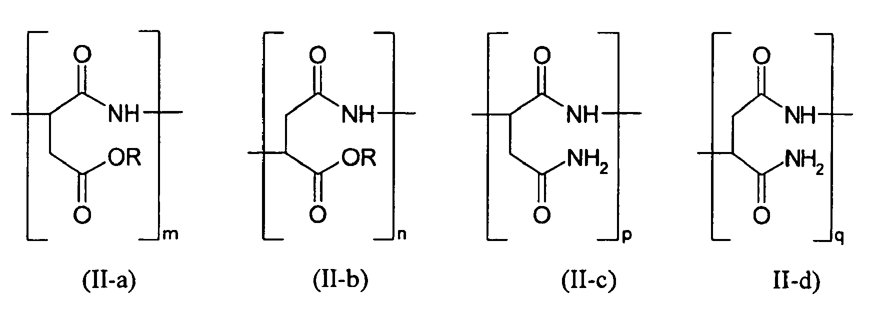 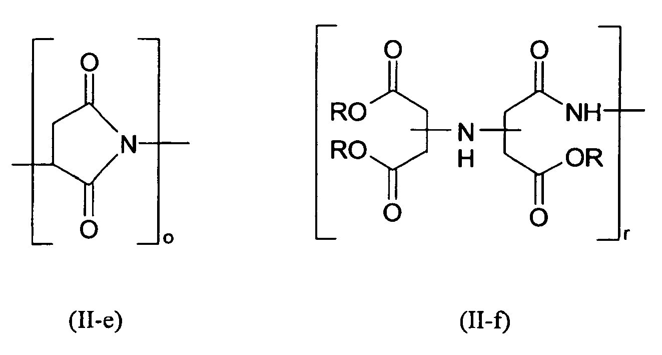 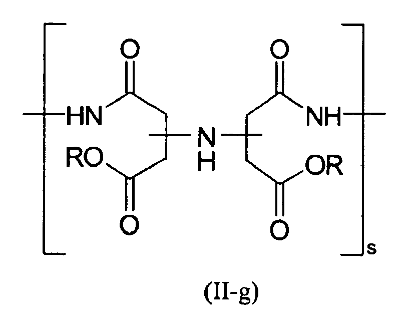 wherein:
each R independently represents hydrogen or a suitable cation;
m, n, o, p, q, r and s independently from one another are 0 or an integer up to 300, whereby the sum of m + n +o + p +q + r + s is in the range of 4 to 300.

2. A composition according to claim 1 wherein
p and q independently are 0 or an integer from 1 to 10;
r is 0 or the integer 1 or 2;
s is 0 or an integer from 1 to 10.

3. A composition according to any of claims 1 or 2 wherein part of the monomeric units of the sequestering agent is replaced by analogues such as:
malic acid units of formula 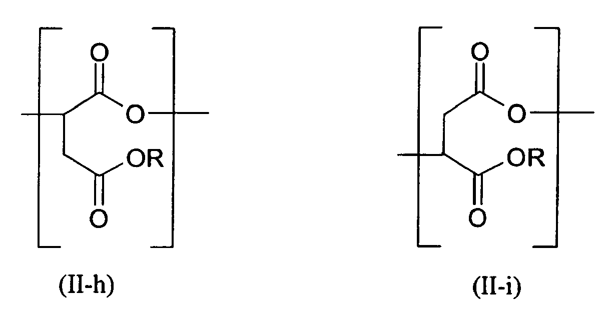
wherein R is as defined above;
maleic acid and and fumaric acid units of formula

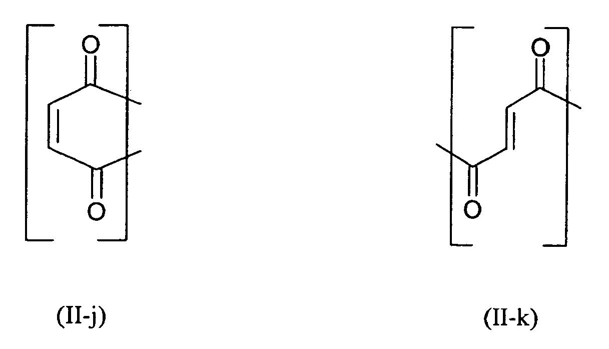

4. A composition according to claim 3 wherein up to 10 monomeric units per polymeric molecule may be replaced by any of the malic, maleic or fumaric residues defined in claim 3.

5. A composition according to any of claims 1 or 2 wherein the polyaspartic acid or its salt-form can be represented by the following formula: 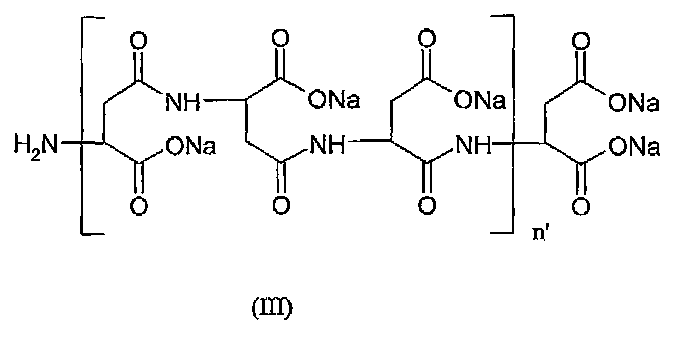 wherein n' is an integer in the range of 1 to 300;
and wherein some or all of the sodium cations may be replaced by one or more cations selected from hydrogen, potassium, ammonium or substituted ammonium.

6. A composition according to claims 1-5, wherein the chelating agent is present in a concentration in the range of 0,1 - 95 % and the sequestering agent is present in a concentration in the range of 0,1 - 45 %, w/w relative to the total weight of the composition.

7. A composition according to claims 1-5, wherein the chelating agent is present in a concentration in the range of 0,4 - 85 % and the sequestering agent in a concentration in the range of 0,2 - 35 %, w/w relative to the total weight of the composition.

8. A composition according to claims 1-5, wherein the weight ratio of the chelating agent to the sequestering agent is in the range between 20 :1 and 1:20, in particular in the range between 10 :1 and 1:10, more in particular in the range between 5 :1 and 1 : 5.

9. A composition according to claims 1-8 further containing an antibacterial, an antiinflammatory or a plant extract.

10. Use of a pharmaceutical composition as claimed in any of claims 1-9 for the manufacture of a medicament for the topical treatment of eczema, irritation or skin dryness.

## Patentansprüche

1. Pharmazeutische Zubereitung, umfassend:
ein kosmetisch oder pharmazeutisch akzeptables Chelatisierungsagens, ausgewählt aus der Gruppe bestehend aus Iminodisuccinat-Salzen und der Säureform davon; und ein kosmetisch oder pharmazeutisch akzeptables Sequestrieragens, ausgewählt aus der Gruppe bestehend aus Polyasparaginsäure und deren Salzen, wobei das Chelatisierungsagens eine Verbindung ist, die strukturell darstellbar ist durch die Formel: 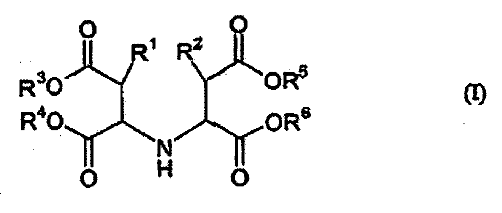
wobei:
R¹ und R² unabhängig voneinander Wasserstoff oder Hydroxy darstellen;
R³, R⁴, R⁵ und R⁶ unabhängig voneinander Wasserstoff oder ein Alkalimetall- oder Ammonium- oder substituiertes Ammonium-Kation darstellen;
und das Sequestrieragens ein aus einer oder mehreren Asparaginsäure-Einheit(en) aufgebautes Polymer ist, das durch die folgenden Strukturformeln darstellbar ist: 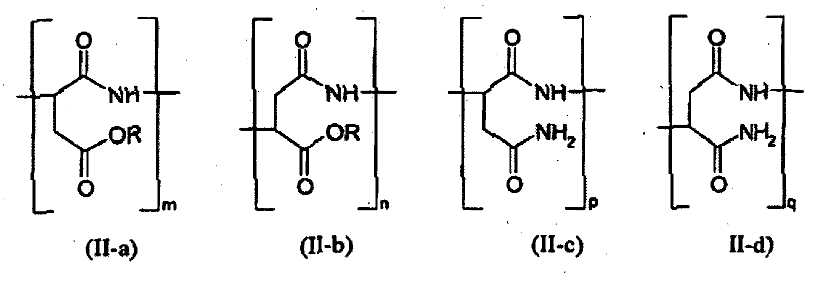 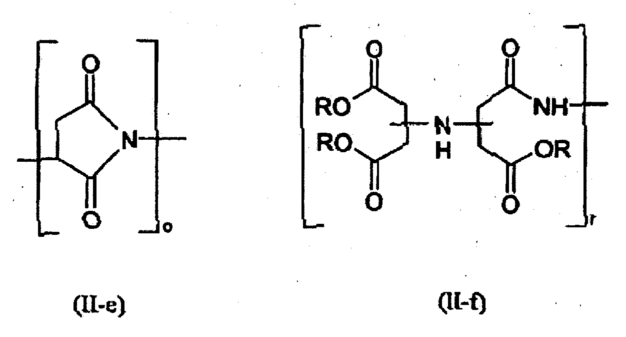 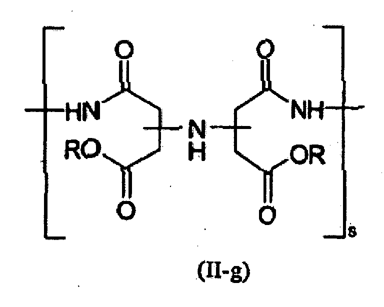 wobei:
jedes R unabhängig Wasserstoff oder ein geeignetes Kation darstellt;
m, n, o, p, q, r und s unabhängig voneinander 0 oder eine ganze Zahl bis 300 sind, wobei die Summe von m + n + o + p + q + r + s im Bereich von 4 bis 300 liegt.

2. Zubereitung nach Anspruch 1, **dadurch gekennzeichnet, dass**
p und q unabhängig 0 oder eine ganze Zahl von 1 bis 10 sind;
r 0 oder die ganze Zahl 1 oder 2 ist;
s 0 oder eine ganze Zahl von 1 bis 10 ist.

3. Zubereitung nach einem der Ansprüche 1 oder 2, **dadurch gekennzeichnet, dass** ein Teil der monomeren Einheiten des Sequestrieragens durch Analoga ersetzt ist, wie z. B.:
Äpfelsäure-Einheiten gemäß der Formel 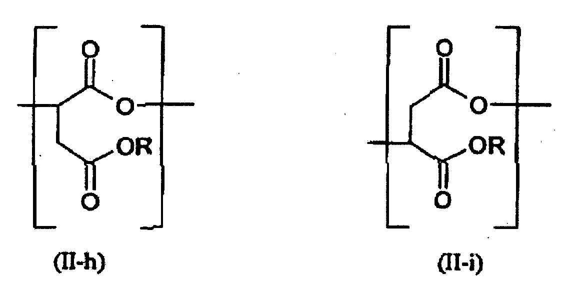
wobei R wie oben definiert ist;
Maleinsäure- und Fumarsäure-Einheiten gemäß der Formel

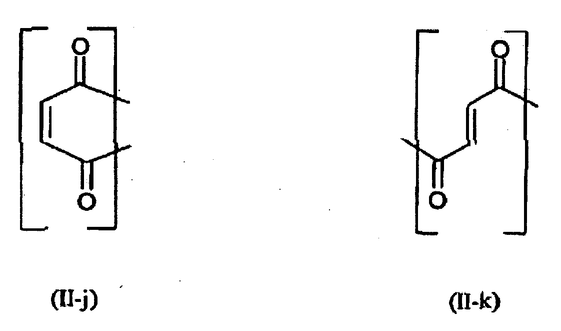

4. Zubereitung nach Anspruch 3, **dadurch gekennzeichnet, dass** bis zu 10 monomere Einheiten pro polymerem Molekül durch einen der in Anspruch 3 definierten Äpfel-, Malein- oder Fumarreste ersetzt sein können.

5. Zubereitung nach einem der Ansprüche 1 oder 2, **dadurch gekennzeichnet, dass** die Polyasparaginsäure oder ihre Salzform durch die folgende Formel darstellbar ist: 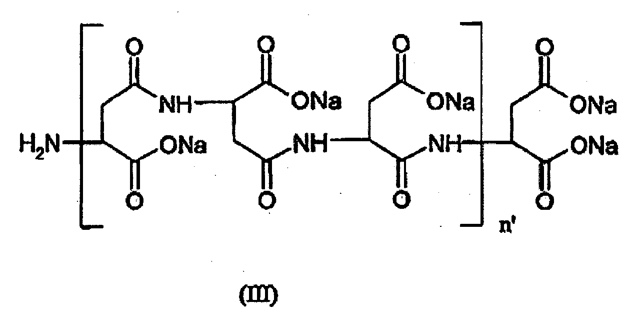 wobei n' eine ganze Zahl im Bereich von 1 bis 300 ist;
und wobei einige oder alle der Natrium-Kationen durch ein oder mehrere Kation(en), ausgewählt aus Wasserstoff, Kalium, Ammonium oder substituiertem Ammonium, ersetzt sein können.

6. Zubereitung nach einem der Ansprüche 1 bis 5, **dadurch gekennzeichnet, dass** das Chelatisierungsagens in einer Konzentration im Bereich von 0,1-95 % und das Sequestrieragens in einer Konzentration im Bereich von 0,1-45 %, Masseprozent bezogen auf das Gesamtgewicht der Zubereitung, vorhanden ist.

7. Zubereitung nach einem der Ansprüche 1 bis 5, **dadurch gekennzeichnet, dass** das Chelatisierungsagens in einer Konzentration im Bereich von 0,4-85 % und das Sequestrieragens in einer Konzentration im Bereich von 0,2-35 %, Masseprozent bezogen auf das Gesamtgewicht der Zubereitung, vorhanden ist.

8. Zubereitung nach einem der Ansprüche 1 bis 5, **dadurch gekennzeichnet, dass** das Gewichtsverhältnis des Chelatisierungsagens zum Sequestrieragens im Bereich zwischen 20 : 1 und 1 : 20, insbesondere im Bereich zwischen 10 : 1 und 1 : 10, weiter insbesondere im Bereich zwischen 5 : 1 und 1 : 5, liegt.

9. Zubereitung nach einem der Ansprüche 1 bis 8, ferner enthaltend ein antibakterielles Mittel, ein entzündungshemmendes Mittel oder einen Pflanzenextrakt.

10. Verwendung einer pharmazeutischen Zubereitung nach einem der Ansprüche 1 bis 9 zur Herstellung eines Medikaments zur topischen Behandlung von Ekzem, Reizung oder Hauttrockenheit.

## Revendications

1. Composition pharmaceutique comprenant :
un agent chélatant cosmétiquement ou pharmaceutiquement acceptable, choisi dans le groupe constitué par les sels d'iminodisuccinate et leur forme acide ; et un agent séquestrant cosmétiquement ou pharmaceutiquement acceptable, choisi dans le groupe constitué par l'acide polyaspartique et ses sels, dans laquelle l'agent chélatant est un composé qui peut être structurellement représenté par la formule : 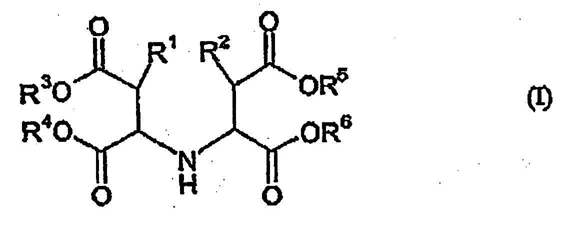
dans laquelle
R¹ et R², représentent indépendamment l'un de l'autre, un hydrogène ou un hydroxy ;
R ³ R⁴, R⁵ et R⁶, représentent indépendamment l'un de l'autre, un hydrogène ou un métal alcalin ou un cation ammonium ou ammonium substitué ;
et l'agent séquestrant est un polymère constitué d'un ou de plusieurs motifs d'acide aspartique qui peuvent être représentés par les formules structurelles suivantes : 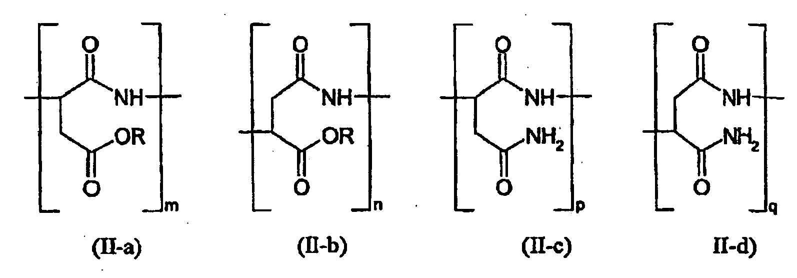 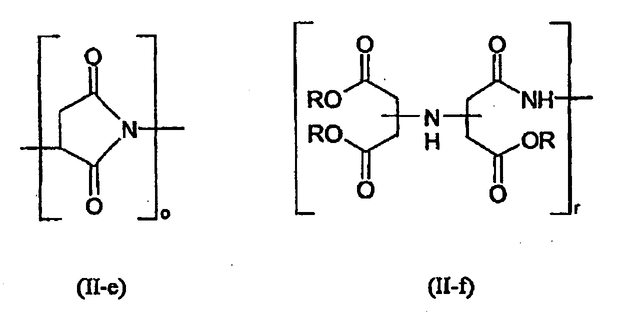 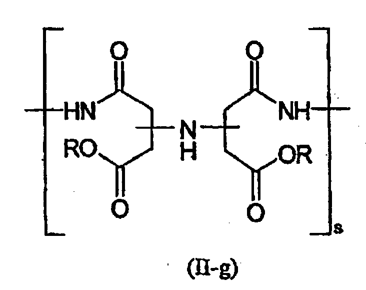 dans lesquelles :
chaque R, indépendamment, représente l'hydrogène ou un cation approprié ;
m, n, o, p, q, r et s, indépendamment les uns des autres, sont 0 ou un entier allant jusqu'à 300, où la somme de m + n + o + p + q + r + s est située dans la plage de 4 à 300.

2. Composition selon la revendication 1, dans laquelle
p et q, indépendamment, sont 0 ou un entier de 1 à 10 ;
r est 0 ou l'entier 1 ou 2 ;
s est 0 ou un entier de 1 à 10.

3. Composition selon l'une quelconque des revendications 1 ou 2, dans laquelle une partie des motifs monomères de l'agent séquestrant est remplacée par des analogues tels que :
des motifs d'acide malique de formule : 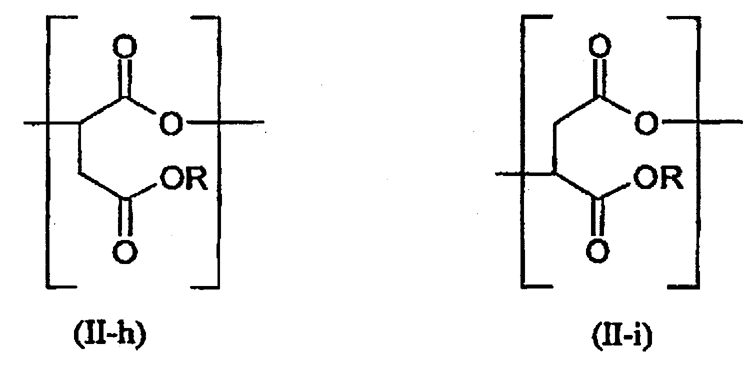
dans laquelle R est tel que défini ci-dessus ;
des motifs d'acide maléique et d'acide fumarique de formule

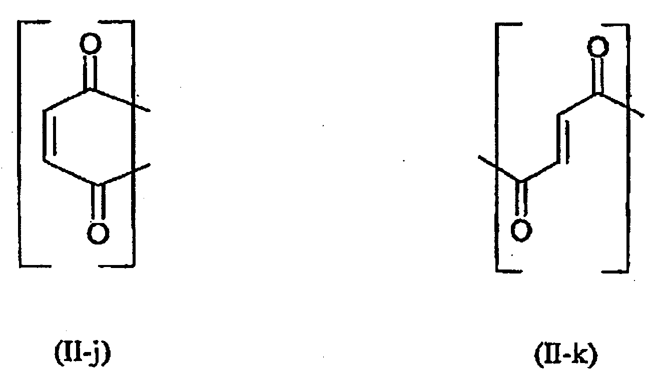

4. Composition selon la revendication 3, dans laquelle jusqu'à 10 motifs monomères par molécule de polymère peuvent être remplacés par un quelconque des résidus d'acide malique, maléique ou fumarique définis dans la revendication 3.

5. Composition selon l'une quelconque des revendications 1 ou 2, dans laquelle l'acide polyaspartique ou sa forme saline peut être représenté par la formule suivante: 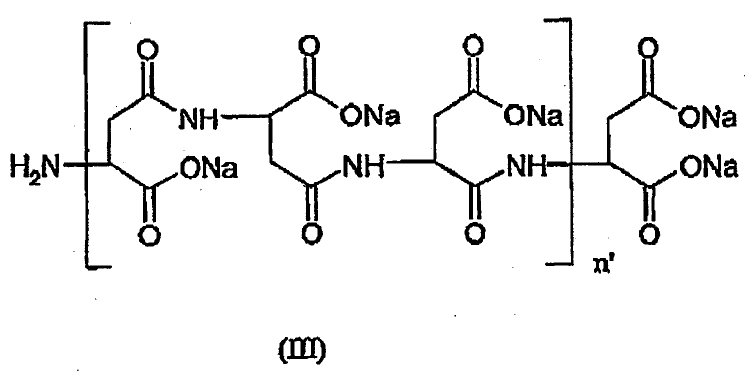 dans laquelle n' est un entier dans la plage de 1 à 300 ;
et dans laquelle certains ou tous les cations de sodium peuvent être remplacés par un ou plusieurs cations choisis parmi l'hydrogène, le potassium, l'ammonium ou l'ammonium substitué.

6. Composition selon les revendications 1 à 5, dans laquelle l'agent chélatant est présent en une concentration dans la plage allant de 0,1 à 95 % et l'agent séquestrant est présent en une concentration dans la plage allant de 0,1 à 45 % en poids/poids par rapport au poids total de la composition.

7. Composition selon les revendications 1 à 5, dans laquelle l'agent chélatant est présent en une concentration dans la plage allant de 0,4 à 85 % et l'agent séquestrant est présent en une concentration dans la plage allant de 0,2 à 35 % en poids/poids par rapport au poids total de la composition.

8. Composition selon les revendications 1 à 5, dans laquelle le rapport en poids entre l'agent chélatant et l'agent séquestrant est dans la plage entre 20/1 et 1/20, en particulier dans la plage entre 10/1 et 1/10, plus particulièrement dans la plage entre 5/1 et 1/5.

9. Composition selon les revendications 1 à 8, contenant en outre un agent antibactérien, anti-inflammatoire ou un extrait de plante.

10. Utilisation d'une composition pharmaceutique selon l'une quelconque des revendications 1 à 9, pour la fabrication d'un médicament pour le traitement topique de l'eczéma, de l'irritation ou de la sécheresse de la peau.
